# EUROPÄISCHE PATENTSCHRIFT

(11) **EP 1 689 734 B1**
(45) Veröffentlichungstag und Bekanntmachung des Hinweises auf die Patenterteilung: **23.07.2008**
(21) Anmeldenummer: 04819579.6
(22) Anmeldetag: 05.11.2004
(51) Int. Cl.: C07D 311/30

(54) **FLAVONOID-DERIVAT**
FLAVONOID DERIVATIVE
DERIVE DE FLAVONOIDE

(30) Priorität: 05.12.2003 DE 10357004
(43) Veröffentlichungstag der Anmeldung: 16.08.2006
(73) Patentinhaber: Merck Patent GmbH, 64293 Darmstadt (DE)
(72) Erfinder: BUCHHOLZ, Herwig, 60599 Frankfurt (DE); WIRTH, Corinna, 64285 Darmstadt (DE); CAROLA, Christophe, 69120 Heidelberg (DE); ALVES FONTES, Rosane, 21825-100 Rio de Janeiro (BR)
(86) Internationale Anmeldenummer: PCT/EP2004/012538
(87) Internationale Veröffentlichungsnummer: WO 2005/054222

(56) Entgegenhaltungen:
- CHEN ET AL.: "New Flavoid Glycosides of Helicteres Angustifolia" HETEROCYCLES, Bd. 38, Nr. 6, 1994, Seiten 1399-1406, XP009048050
- RAMESH ET AL.: "A new Flavone Methyl Ether from Helicteres Isora" J.NAT.PRODUCTS, Bd. 58, Nr. 8, 1995, Seiten 1242-1243, XP009048065

## Beschreibung

Die Erfindung betrifft ein neues Flavonoid-Derivat, ein Extrakt enthaltend das Flavonoid-Derivat, deren kosmetische und pharmazeutische Verwendung sowie zur Verwendung als Nahrungsergänzungsmittel, das Flavonoid-Derivat bzw. den Extrakt enthaltende Zubereitungen sowie ein Verfahren zur Herstellung des Flavonoid-Derivats bzw. des Extrakts.

Die menschliche Haut unterliegt Alterungsprozessen, die teilweise auf intrinsische Prozesse (chrono-ageing) und teilweise auf exogene Faktoren (environmental, z.B. photo-ageing) zurückzuführen sind. Zusätzlich können vorübergehende oder auch andauernde Veränderungen des Hautbildes auftreten wie z. B. Akne, tätige oder trockene Haut, Keratosen, Rosaceae, wie empfindliche, entzündliche, erythematöse, allergische oder autoimmunreaktive Reaktion wie Dermatosen und Photodermatosen.

Zu den exogenen Faktoren zählen insbesondere das Sonnenlicht oder künstliche Strahlenquellen mit vergleichbarem Spektrum sowie Verbindungen, die durch Strahlung entstehen können, wie undefinierte reaktive Photoprodukte, die auch radikalisch oder ionisch sein können. Zu diesen Faktoren zählen auch Zigarettenrauch und die darin enthaltenen reaktiven Verbindungen wie Ozon, freie Radikale, beispielsweise das Hydroxylradikal, Sigulettsauerstoff und andere reaktive Sauerstoff- und Stickstoffverbindungen, die die natürliche Physiologie oder Morphologie der Haut stören.

Zum Schutz der Haut gegenüber Lichtexposition können kosmetische und/oder pharmazeutische Produkte verwendet werden, die UV-Filter enthalten. Vorteilhaft sind dabei insbesondere Wirkstoffe, die neben einem UV-Schutz auch antioxidativ wirken und die Haut somit sowohl durch Verminderung der Lichtexposition als auch durch Inaktivierung von durch Strahlenexposition induzierten oder in sonstiger Weise gebildeten freien Radikalen entgegenwirken. Als besonders geeignet haben sich hierbei Flavonoide erwiesen und hierbei insbesondere Quercetin.

Flavonoide weisen jedoch in der Regel nur eine geringe Wasserlöslichkeit auf und können daher in wässrige Formulierungen oft nur in unzureichenden Mengen eingearbeitet werden. So weist beispielsweise Quercetin eine Löslichkeit in Wasser von nur 0,04 g/l auf.

Hautalterung geht mit einer Verringerung der Schichtdicken der beiden aufeinander liegenden Hautschichten Epidermis und Dermis einher und es wird angenommen, dass dies zumindest teilweise für die Bildung von Falten in der alternden Haut verantwortlich ist. Während die oben liegende Epidermis der Haut vor allem Widerstandsfähigkeit verleiht und die Hauptbarriere ausbildet, verleiht die darunter liegende Dermis der Haut Stärke, Elastizität und Dicke.
Die Epidermis besteht hauptsächlich aus Keratinozyten, die in vier unterschiedliche Differenzierungsstadien unterteilt werden können. Die epidermale Differenzierung ist sehr wichtig für die Ausbildung der essenziellen Hautfunktionen, nämlich als Schutzbarriere gegenüber der Umwelt sowie zur Verhinderung von Wasserverlust vom Körper. In der letzten Stufe der epidermale Differenzierung werden die verhornten Zellhüllen (cornified cell envelope) ausgebildet. Dabei kommt es unter Einwirkung der Transglutaminase zu einer Quervernetzung der Proteine Loricrin, kleiner Prolin-reicher Proteine und Involucrin. Aktivierung der Transglutaminase ist daher auch ein vielversprechenden Ansatz zur Verbesserung der Hautstruktur und zur Bekämpfung der Hautalterung (anti-aging).

Es war Aufgabe der Erfindung neue Verbindungen mit wertvollen Eigenschaften aufzufinden, insbesondere solche, die zur Verwendung in der Kosmetik, der Pharmazie sowie zur Nahrungsergänzung geeignet sind.

Gegenstand der Erfindung ist ein Flavonoid-Derivat mit der chemischen Bezeichnung [5-Hydroxy-7-methoxy-2-(4'-methoxy-phenyl)-4-oxo-4H-chromen-8yl-]-sulfonsäuremonoester, dessen physiologisch unbedenkliche Salze und Solvate. Die Verbindung ist nachfolgend als Strukturformel dargestellt:

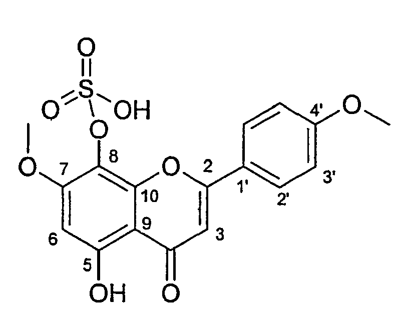

Geeignete Salze sind alle physiologisch verträglichen Metallsalze, insbesondere Alkalimetallsalze, wie z.B. das Natrium- oder das Kaliumsalz, oder Erdalkalimetallsalze, wie z.B. das Magnesium- oder das Calciumsalz sowie das Ammoniumsalz.
Unter Solvaten von [5-Hydroxy-7-methoxy-2-(4'-methoxy-phenyl)-4-oxo-4H-chromen-8yl-]-sulfonsäuremonoester werden Anlagerungen von inerten Lösungsmittelmolekülen an [5-Hydroxy-7-methoxy-2-(4'-methoxy-phenyl)-4-oxo-4H-chromen-8yl-]-sulfonsäuremonoester verstanden, die sich aufgrund ihrer gegenseitigen Anziehungskraft ausbilden. Solvate sind z.B. Mono- oder Dihydrate oder Additionsverbindungen mit Alkoholen, wie z.B. mit Methanol oder Ethanol.

Vor- und nachstehende Ausführungen zu [5-Hydroxy-7-methoxy-2-(4'-methoxy-phenyl)-4-oxo-4H-chromen-8yl-]-sulfonsäuremonoester gelten entsprechend auch für dessen physiologisch unbedenklichen Salze und Solvate.

[5-Hydroxy-7-methoxy-2-(4'-methoxy-phenyl)-4-oxo-4H-chromen-8yl-]-sulfonsäuremonoester absorbiert in einem sehr breiten Bereich ultraviolette Strahlung und weist dabei gleichzeitig eine ausgezeichnete Wasserlöslichkeit (21 g/l bei 25°C) auf. Die Verbindung ist daher besonders geeignet zur Verwendung als UV-Filter und kann in einfacher Weise und in großer Menge in kosmetische und/oder pharmazeutische Präparate, Nahrungsmittel und Nahrungsergänzungsmittel eingearbeitet werden.

Überraschenderweise führt [5-Hydroxy-7-methoxy-2-(4'-methoxy-phenyl)-4-oxo-4H-chromen-8yl-]-sulfonsäuremonoester auch zu einer Induktion der Genexpression von Transglutaminase. Wie oben beschrieben, ist die Transglutaminase maßgeblich am Aufbau eines die Hornzellen umgebenden speziellen Mantels, dem so genannten "cornified cell envelope" beteiligt, der in der Kömerschicht aufgebaut wird und die ursprüngliche Membran in der Hornschicht ersetzt. Dies führt über eine bessere Verankerung der Zellen zu einer Verstärkung der Hautbarriere und damit zu einer Erhöhung der Widerstandskraft der Haut gegenüber Umwelteinflüssen, wie beispielsweise Austrocknung.

Weiterhin wurde gefunden, dass [5-Hydroxy-7-methoxy-2-(4'-methoxyphenyl)-4-oxo-4H-chromen-8yl-]-sulfonsäuremonoester auch die Synthese des Tissue Inhibitor of Metalloproteinase 1 Precursor (TIMP1 ) induziert, was zu einer Hemmung der Metalloproteinase führt, die beispielsweise für die frühzeitige Hautalterung von Rauchern verantwortlich gemacht wird. Auch hierdurch kommt [5-Hydroxy-7-methoxy-2-(4'-methoxy-phenyl)-4-oxo-4H-chromen-8yl-]-sulfonsäuremonoester eine "anti-ageing" Wirkung zu.

[5-Hydroxy-7-methoxy-2-(4'-methoxy-phenyl)-4-oxo-4H-chromen-8yl-]-sulfonsäuremonoester induziert weiterhin die Expression vom Epican-Gen (Hyaluronsäurerezeptor), das eine wichtige Rolle bei der Differenzierung von Keratinozyten spielt, und führt so zu einer Verbesserung der Hautstruktur. Insbesondere führt es zu einer Bildung von glatter Haut.

Die Beeinflussung der Expression der genannten Gene kann mittels cDNA Array nachgewiesen werden.

Weiterhin hemmt [5-Hydroxy-7-methoxy-2-(4'-methoxy-phenyl)-4-oxo-4H-chromen-8yl-]-sulfonsäuremonoester die Arachidonsäurekaskade und ist damit auch antientzündlich wirksam.

[5-Hydroxy-7-methoxy-2-(4'-methoxy-phenyl)-4-oxo-4H-chromen-8yl-]-sulfonsäuremonoester kann vorteilhaft durch Extraktion aus verschiedenen Pflanzen erfolgen. Geeignete Pflanzen, die [5-Hydroxy-7-methoxy-2-(4'-methoxy-phenyl)-4-oxo-4H-chromen-8yl-]-sulfonsäuremonoester enthalten sind Sidastrum acuminatum, Sidastrum burrerense, Sidastrum E.G. Baker, Sidastrum kicranthum, Sidastrum lodiegense, Sidastrum multiflorum, Sidastrum micranthum, Sidastrum paniculatum, Sidastrum strictum, Sidastrum tehuacanum, Sidastrum quinquenervium. Besonders bevorzugt erfolgt die Gewinnung aus Sidastrum micranthum.

Neben [5-Hydroxy-7-methoxy-2-(4'-methoxy-phenyl)-4-oxo-4H-chromen-8yl-]-sulfonsäuremonoester selbst weist auch der bei dessen Gewinnung aus Pflanzenmaterial entstehende Extrakt wertvolle Eigenschaften auf und kann als pharmazeutisches, kosmetisches Nahrungs- und/oder Nahrungsergänzungsmittel-Mittel verwendet werden. Gegenstand der Erfindung ist daher auch der Extrakt enthaltend [5-Hydroxy-7-methoxy-2-(4'-methoxy-phenyl)-4-oxo-4H-chromen-8yl-]-sulfonsäuremonoester erhältlich durch Extraktion von Pflanzenmaterial, ausgewählt aus Sidastrum acuminatum, Sidastrum burrerense, Sidastrum E.G. Baker, Sidastrum kicranthum, Sidastrum lodiegense, Sidastrum multiflorum, Sidastrum micranthum, Sidastrum paniculatum, Sidastrum strictum, Sidastrum tehuacanum oder Sidastrum quinquenervium. Besonders bevorzugt ist dabei ein Extrakt aus Sidastrum micranthum.

Die Herstellung des Pflanzenextrakts erfolgt durch übliche Methoden der Extraktion der Pflanzen bzw. Pflanzenteile. Geeignete Extraktionsverfahren können sein: Mazeration, Remazeration, Digestion, Bewegungsmazeration, Wirbelextraktion, Ultraschallextraktion, Gegenstromextraktion, Perkolation, Reperkolation, Evakolation, Diakolation oder Festflüssig-Extraktion unter kontinuierlichem Rückfluss, die in einem Soxhlet-Extraktor durchgeführt wird.

Extrahiert werden alle Teile der Pflanze, bevorzugt die oberirdischen Teile, besonders bevorzugt die Blätter der Pflanze.

Als Lösungsmittel für die Extraktion kann beispielsweise Wasser oder ein Alkohol verwendet werden.

Gegenstand der Erfindung ist auch ein Verfahren zur Herstellung von [5-Hydroxy-7-methoxy-2-(4'-methoxy-pheny)-4-oxo-4H-chromen-8yl-]-sulfonsäuremonoester durch Extraktion von Pflanzenmaterial der Gattungen Sidastrum acuminatum, Sidastrum-burrerense, Sidastrum E.G. Baker, Sidastrum-kicranthum, Sidastrum lodiegense, Sidastrum multiflorum, Sidastrum micranthum, Sidastrum paniculatum, Sidastrum strictum, Sidastrum tehuacanum oder Sidastrum quinquenervium. Bevorzugt findet als Pflanzenmaterial Sidastrum micranthum Verwendung.

Es ist dem allgemeinen Wissen des Fachmanns zuzurechnen, wie diese Extraktionen im Einzelnen durchgeführt werden und der erhaltene Rohextrakt durch allgemein geläufige Methoden aufgereinigt werden können.

Der erfindungsgemäße Extrakt umfasst auch Pflanzenextrakte, die nach der Extraktion einer weiteren Aufarbeitung unterzogen worden sind, um beispielsweise unerwünschte Begleitstoffe abzutrennen oder die gewünschten Inhaltsstoffe anzureichern.

Der erfindungsgemäße Extrakt kann [5-Hydroxy-7-methoxy-2-(4'-methoxyphenyl)-4-oxo-4H-chromen-8yl-]-sulfonsäuremonoester in einer Menge von 0,1 bis 100 Gew.-% enthalten. Nach einer bevorzugten Ausführungsform enthält der Extrakt 5 bis 100 Gew.-%, besonders bevorzugt 30 bis 100 Gew.-%, ganz besonders bevorzugt 90 bis 100 Gew.-% [5-Hydroxy-7-methoxy-2-(4'-methoxy-phenyl)-4-oxo-4H-chromen-8yl-]-sulfonsäuremonoester.

Auf Grund der genannten Wirkungen und Eigenschaften sind [5-Hydroxy-7-methoxy-2-(4'-methoxy-phenyl)-4-oxo-4H-chromen-8yl-]-sulfonsäuremonoester und der diese Verbindungen enthaltende Extrakt hervorragend als Inhaltsstoff von Zubereitungen geeignet, die innerlich und/oder äußerlich anwendbar sind, beispielsweise als Arzneimittel; kosmetische, Nahrungs- und/oder als Nahrungsergänzungsmittel. Gegenstand der Erfindung sind daher Zubereitungen mindestens enthaltend [5-Hydroxy-7-methoxy-2-(4'-methoxy-phenyl)-4-oxo-4H-chromen-8yl-]-sulfonsäuremonoester und/oder ein Extrakt aus den oben genannten Pflanzen sowie gegebenenfalls Träger- und/oder Hilfsstoffe.

Unter Zubereitung wird eine Formulierung verstanden, die den Wirkstoff und/oder den Extrakt enthält und zur Anwendung am Mensch oder Tier bestimmt ist, beispielsweise durch Auftragen auf die Haut, zur oralen Einnahme, Inhalation, Infusion oder Injektion. Je nach Art der Zubereitung kann die Zubereitung neben dem Wirkstoff/Extrakt Träger- und/oder Hilfsstoffe enthalten, sie kann aber auch ausschließlich aus dem Wirkstoff/Extrakt selbst bestehen, beispielsweise in Form eines Pulvers, das z.B. direkt oral eingenommen oder inhaliert werden kann. Neben der erfindungsgemäßen Verbindung bzw. dem erfindungsgemäßen Extrakt kann die Zubereitung auch weitere Wirkstoffe enthalten.

Bei Extraktion aus Pflanzenmaterial kann [5-Hydroxy-7-methoxy-2-(4'-methoxy-phenyl)-4-oxo-4H-chromen-8yl-]-sulfonsäuremonoester entweder in isolierter Form oder auch in nicht isolierter Form weiterverarbeitet werden, also z.B. in Form eines Extrakts oder in Form eines aufgereinigten Extrakts oder auch in Form der aus dem Pflanzenextrakt hergestellten Reinsubstanz in Zubereitungen eingearbeitet werden.

Bevorzugt enthält die Zubereitung [5-Hydroxy-7-methoxy-2-(4'-methoxyphenyl)-4.-oxo-4H-chromen-8yl-]-sulfonsäuremonoester in der Form eines Extrakts, eines aufgereinigten Extrakts oder in Form der aus dem Extrakt hergestellten Reinsubstanz.

Gegenstand der Erfindung ist auch die Verwendung von [5-Hydroxy-7-methoxy-2-(4'-methoxy-phenyl)-4-oxo-4H-chromen-8yl-]-sulfonsäuremonoester bzw. des Extrakts zur Herstellung der Zubereitung, die **dadurch gekennzeichnet** ist, dass diese eine pharmazeutische Zubereitung ist. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Nach einer bevorzugten Ausführungsform ist die erfindungsgemäße Zubereitung **dadurch gekennzeichnet**, dass diese ein Arzneimittel ist.

Die Arzneimittel können in der Human- oder Veterinärmedizin verwendet werden. Als Trägerstoffe kommen organische oder anorganische Substanzen in Frage, die sich für die enterale (z.B. orale), parenterale oder topische Applikation eignen und mit [5-Hydroxy-7-methoxy-2-(4'-methoxyphenyl)-4-oxo-4H-chromen-8yl-]-sulfonsäuremonoester bzw. dem Extrakt nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die Pflanzenextrakte können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder mehrere weitere Wirkstoffe enthalten, z.B. ein oder mehrere Vitamine.

[5-Hydroxy-7-methoxy-2-(4'-methoxy-phenyl)-4-oxo-4H-chromen-8yl-]-sulfonsäuremonoester bzw. der Extrakt wird in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt.

Die pharmazeutischen Formulierungen enthaltend einen oder mehrere Pflanzenextrakt/e können mit Hilfe von Techniken hergestellt werden, die dem Fachmann wohl bekannt sind.

Nach einer weiteren bevorzugten Ausführungsform ist die Zubereitung **dadurch gekennzeichnet**, dass diese ein kosmetisches Mittel ist.

Besonders bevorzugt ist eine Zubereitung, die **dadurch gekennzeichnet** ist, dass sie ein Hautbehandlungsmittel ist.

Ein Hautbehandlungsmittel ist eine kosmetische, dermatologische oder pharmazeutische Zubereitung, die sich zur topischen Anwendung eignet. Typischerweise enthält die Zubereitung dabei übliche, hautvertragliche und entsprechend des Verwendungszweckes getestete Träger und ggf. weitere Hilfsmittel und Wirkstoffe.

Ein weiterer Gegenstand der vorliegenden Anmeldung ist daher eine Zubereitung zur topischen Anwendung umfassend
a) [5-Hydroxy-7-methoxy-2-(4'-methoxy-phenyl)-4-oxo-4H-chromen-8yl-]-sulfonsäuremonoester und/oder einen Extrakt, wie oben beschrieben,
b) einen hautverträglichen Träger, und
c) optional einen oder mehrere weitere Wirkstoffe mit hautpflegender und/oder entzündungshemmender Wirkung.

Unter anderem auf Grund der bereits genannten UV-Absorption von [5-Hydroxy-7-methoxy-2-(4'-methöxy-phenyt)-4-oxo-4H-chromen-8yl-]-sulfonsäuremonoester eignet sich eine diese Verbindung und/oder den Extrakt enthaltende Zubereitung auch zum Schutz menschlicher Haut bzw. zum Schutz von Körperzellen gegen oxidativen Stress, d.h. z.B. gegen Schädigungen durch Radikale, wie sie durch Sonneneinstrahlung erzeugt werden. Der Schutz gegenüber ultraviolette Strahlung durch die erfindungsgemäße Zubereitung kann durch Einarbeitung eines oder mehrerer weiteren UV-Filters noch verstärkt werden.

Gegenstand der Erfindung ist daher auch eine Zubereitung, die **dadurch gekennzeichnet** ist, dass sie weiterhin einen oder mehrere UV-Filter enthält.

Prinzipiell kommen alle UV-Filter für eine Kombination in Frage. Besonders bevorzugt sind solche UV-Filter, deren physiologische Unbedenklichkeit bereits nachgewiesen ist. Sowohl für UV-A- als auch UVB-Filter gibt es viele aus der Fachliteratur bekannte und bewährte Substanzen, z.B.

Benzylidenkampferderivate wie
- 3-(4'-Methylbenzyliden)-dl-kampfer (z.B. Eusolex^{®} 6300),
- 3-Benzylidenkampfer (z.B. Mexoryl^{®} SD),
- Polymere von N-{(2 und 4)-[(2-oxoborn-3-yliden)methyl]benzyl}-acrylamid (z.B. Mexoryl^{®} SW),
- N,N,N-Trimethyl-4-(2-oxoborn-3-ylidenmethyl)anilinium methylsulfat (z.B. Mexoryl^{®} SK) oder
- α-(2-Oxoborn-3-yliden)toluol-4-sulfonsäure (z.B. Mexoryl^{®} SL),

Benzoyl- oder Dibenzoylmethane wie
- 1-(4-tert-Butylphenyl)-3-(4-methoxyphenyl)propan-1,3-dion (z.B. Eusolex^{®} 9020) oder
- 4-Isopropyldibenzoylmethan (z.B. Eusolex^{®} 8020),

Benzophenone wie
- 2-Hydroxy-4-methoxybenzophenon (z.B. Eusolex^{®} 4360) oder
- 2-Hydroxy-4-methoxybenzophenon-5-sulfonsäure und ihr Natriumsalz (z.B. Uvinul^{®} MS-40),

Methoxyzimtsäureester wie
- Methoxyzimtsäureoctylester (z.B. Eusolex^{®} 2292),
- 4-Methoxyzimtsäureisopentylester, z.B. als Gemisch der Isomere (z.B. Neo Heliopan^{®} E 1000),

Salicylatderivate wie
- 2-Ethylhexylsalicylat (z.B. Eusolex^{®} OS),
- 4-lsopropylbenzylsalicylat (z.B. Megasol^{®}) oder
- 3,3,5-Trimethylcyclohexylsalicylat (z.B. Eusolex^{®} HMS),

4-Aminob.enzoesäure und Derivate wie
- 4-Aminobenzoesäure,
- 4-(Dimethylamino)benzoesäure-2-ethylhexylester (z.B. Eusolek^{®} 6007),
- ethoxylierter 4-Aminobenzoesäureethylester (z.B. Uvinul^{®} P25),

Benzimidazolderivate wie
- 2-Phenylbenzimidazol-5-sulfonsäure sowie ihre Kalium-, Natrium- und Triethanolaminsalze (z.B. Eusolex^{®} 232),
- 2,2'-(1,4-Phenylen)bis-(1H-benzimidazol-4,6-disulfonsäure, Mononatriumsalz) (CAS-Nr. 180 898-37-7),
- 2,2'-(1,4-Phenylen)bis-(1 H-benzimidazol-5-sulfonsäure) sowie ihre Kalium-, Natrium- und Triethanolaminsalze,
und weitere Substanzen wie
- 2-Cyano-3,3-diphenylacrylsäure-2-ethylhexylester (z.B. Eusolex^{®} OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl^{®} SX),
- 2,4,6-Trianilino-(p-carbo-2'-ethylhexyl-1'-oxi)-1,3,5-triazin (z.B. Uvinul^{®} T 150),
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl [und ca. 6% methyl [2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n≈60) (CAS-Nr. 207574-74-1),
- 2,2'-Methylen-bis-(6-(2H-benzotriazol-2-yl)-4-(1,1,3,3-tetramethylbutyl)phenol) (CAS-Nr. 103 597-45-1),
- 2,4-bis-{[4-(2-Ethyl-hexyloxy)-2-hydroxyl]-phenyl}-6-(4-methoxyphenyl)-1,3,5-triazin (CAS-Nr. 103 597-45-,187 393-00-6).

Die in der Liste aufgeführten Verbindungen sind nur als Beispiele aufzufassen. Selbstverständlich können auch andere UV-Filter verwendet werden. Diese organischen UV-Filter werden in der Regel in einer Menge von 0,5 bis 20 Gew.-%, vorzugsweise in einer Menge von 1 bis 15 Gew.-% und insbesondere bevorzugt in Mengen von 2 bis 8 Gew.-% je Einzelsubstanz in die erfindungsgemässen Zubereitungen, insbesondere in kosmetische Formulierungen, eingearbeitet. Soweit die erfindungsgemäße Zubereitung neben 5-Hydroxy-7-methoxy-2-(4'-methoxy-phenyl)-4-oxo-4H-chromen-6yl-]-sulfonsäuremonoester und/oder dem erfindungsgemäßen. Extrakt weitere organische UV-Filter enthält, sind diese bezogen auf das Gesamtgewicht der Formulierung, insbesondere bei kosmetischen Formulierungen, üblicherweise in einer Menge von bis zu 40 Gew.-%, vorzugsweise in einer Menge von 5 bis 25 Gew.-%, enthalten.

Als anorganische UV-Filter sind solche aus der Gruppe der Titandioxide wie z.B. gecoatetes Titandioxid (z.B. Eusolex^{®} T-2000, Eusolex^{®} T-AQUA), Zinkoxide (z.B. Sachtotec^{®}), Eisenoxide oder auch Ceroxide denkbar. Diese anorganischen UV-Filter sind, bezogen auf das Gesamtgewicht der Formulierung, insbesondere bei kosmetischen Formulierungen, in der Regel in einer Menge von 0,5 bis 20 Gew.-%, vorzugsweise in einer Menge von 2 bis 10 Gew.-%, enthalten.

Werden verschiedene anorganische oder organische UV-Filter eingesetzt, so können diese in nahezu beliebigen Verhältnissen zueinander verwendet werden. Üblicherweise liegen die Verhältnisse der einzelnen Substanzen zueinander im Bereich 1:10 - 10:1, vorzugsweise im Bereich 1:5-5:1 und insbesondere bevorzugt im Bereich 1:2-2:1. Werden UV-A- und UV-B-Filter eingesetzt, so ist es für die meisten Anwendungen von Vorteil, wenn der Anteil an UV-B-Filtern überwiegt und das Verhältnis von UV-A-Filtern : UV-B-Filtern im Bereich 1:1 bis 1:10 liegt.

Bevorzugte Verbindungen mit UV-filternden Eigenschaften, die in der erfindungsgemäßen Zubereitung bevorzugt enthalten sein können, insbesondere wenn diese eine kosmetische Zubereitung ist, sind 3-(4'-Methylbenzyliden)-dl-kampfer, 1-(4-tert-Butylphenyl)-3-(4-methoxyphenyl)propan-1,3-dion, 4-lsopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester.

Wie oben beschrieben, haben [5-Hydroxy-7-methoxy-2-(4'-methoxyphenyl)-4-oxo-4H-chromen-8yl-]-sulfonsäuremonoester bzw. der diese Verbindung enthaltende Extrakt u. a. folgende Wirkungen: sie erhöhen die Widerstandskraft der Haut gegenüber Umwelteinflüssen, wie beispielsweise Austrocknung, wirken der Hautalterung entgegen, führen zu einer Verbesserung der Hautstruktur, insbesondere zur Bildung von glatter Haut und sind antientzündlich wirksam. Gegenstand der Erfindung ist daher auch die Verwendung von [5-Hydroxy-7-methoxy-2-(4'-methoxyphenyl)-4-oxo-4H-chromen-8yl-]-sulfonsäuremonoester und/oder des diese Verbindung enthaltenden Extrakts zur Herstellung einer pharmazeutischen und/oder kosmetischen Zubereitung zur Erhöhung der Widerstandskraft der Haut gegenüber Umwelteinflüssen, insbesondere Austrocknung, zur Vermeidung der Hautalterung, zur Verbesserung der Hautstruktur, insbesondere zur Bildung glatter Haut.

[5-Hydroxy-7-methoxy-2-(4'-methoxy-phenyl)-4-oxo-4H-chromen-8yl-]-sulfonsäuremonoester bzw. der diese Verbindung enthaltende Extrakt wirken auch als Radikalfänger und damit oxidativem Stress entgegen. Weiterhin weisen Sie eine anti-allergische und anti-irritative Wirkung auf und können somit zur Behandlung oder vorbeugenden Behandlung von Allergien, Entzündungen und Irritationen, insbesondere der Haut, verwendet werden. Gegenstand der Erfindung ist daher auch die Verwendung von 5-Hydroxy-7-methoxy-2-(4'-methoxy-phenyl)-4-oxo-4H-chromen-6yl-]-sulfonsäuremonoester und/oder des diese Verbindung enthaltenden Extrakts zur Herstellung einer pharmazeutischen und/oder kosmetischen Zubereitung zum Schutz gegen oxidativen Stress sowie zur Bekämpfung von Allergien, Entzündungen und Irritationen. Bevorzugt sind Zubereitungen zur topischen Anwendung auf der Haut.

Damit [5-Hydroxy-7-methoxy-2-(4'-methoxy-phenyl)-4-oxo-4H-chromen-8yl-]-sulfonsäuremonoester bzw. der diese Verbindung enthaltende Extrakt ihre positive Wirkung als Radikalfänger auf die Haut besonders gut entwickeln können, kann es vorteilhaft sein, diese in tiefere Hautschichten eindringen zu lassen. Soweit die Eindringtiefe in epidermale Schichten nicht ausreichend ist, kann diese durch geeignete Transportmittel, beispielsweise Liposomen, erhöht werden, die einen Transport der der Verbindung durch die äußeren Hautschichten ermöglichen. Schließlich ist auch ein systemischer Transport von [5-Hydroxy-7-methoxy-2-(4'-methoxyphenyl)-4-oxo-4H-chromen-8yl-]-sulfonsäuremonoester denkbar. Die Zubereitung wird dann beispielsweise so gestaltet, dass sie für eine orale Gabe geeignet ist.

Wie bereits erwähnt, wirken [5-Hydroxy-7-methoxy-2-(4'-methoxy-phenyl)-4-oxo-4H-chromen-8yl-]-sulfonsäuremonoester bzw. der diese Verbindung enthaltende Extrakt als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (11)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Aufgrund ihrer Wirkungen eignet sich [5-Hydroxy-7-methoxy-2-(4'-methoxy-phenyl)-4-oxo-4H-chromen-8yl-]-sulfonsäuremonoester bzw. der diese Verbindung enthaltende Extrakt auch zur Herstellung von Zubereitungen zur Immunprotektion und zum Schutz der DNA und RNA. Die erhaltenen Zubereitungen eignen sich insbesondere zum Schutz von DNA und RNA vor oxidativen Angriffen, vor Radikalen und vor Schädigung durch Strahlung, insbesondere UV-Strahlung. Soweit die Verbindung/der Extrakt in Form von Zubereitungen zur Anwendung auf der Haut verwendet werden, ergibt sich gegenüber UV-Strahlung eine doppelte Schutzwirkung: durch die Absorption von UV-Strahlung, die deren Einwirkung auf die Haut verhindert, sowie durch die Wirkung als Radikalfänger, die den durch dennoch eindringende UV-Strahlung induzierten Radikalen entgegengewirkt.

Weiterhin eignet sich [5-Hydroxy-7-methoxy-2-(4'-methoxy-phenyl)-4-oxo-4H-chromen-8yl-]-sulfonsäuremonoester bzw. der diese Verbindung enthaltende Extrakt zur Herstellung von Zubereitungen zum Zellschutz, insbesondere zum Schutz von Langerhans-Zellen vor Schäden durch die oben genannten Einflüsse. Ausdrücklicher Gegenstand der vorliegenden Erfindung ist daher auch die Verwendung einer Zubereitung, die [5-Hydroxy-7-methoxy-2-(4'-methoxy-phenyl)-4-oxo-4H-chromen-8yl-]-sulfonsäuremonoester und/oder den diese Verbindung enthaltenden Extrakt enthalten, zu den genannten Zwecken.

Die erfindungsgemäße Zubereitung eignet sich auch zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonicá, der polymorphen Akne, der Akne rosaceae, der nodulären Akne, der Akne conglobata, der altersbedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Haut-atopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillo-matosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der licht-bedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körper-bereiche mit einer immuno-logischen Komponente, zur Behandlung von Herz-/Kreislauf Erkran-kungen, wie Arteriosklerose oder Bluthochdruck, sowie des Insulinunabhängigen Diabetes, zur Behandlung von Haut-problemen, die durch UV-Strahlung hervorgerufen werden.

Die schützende Wirkung gegen oxidativen Stress bzw. gegen die Einwirkung von Radikalen kann weiter verbessert werden, wenn die erfindungsgemässe Zubereitung ein oder mehrere weitere Antioxidantien enthält. Weiterer Gegenstand der Erfindung ist daher eine Zubereitung, die **dadurch gekennzeichnet** ist, dass sie ein oder mehrere weitere/s Antioxidans/Antioxidantien enthält.

Es gibt viele aus der Fachliteratur bekannte und bewährte Substanzen, die als Antioxidantien enthalten sein können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B.

Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in der erfindungsgemässen Zubereitung geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004),

Bevorzugt enthält die erfindungsgemäße Zubereitung als weitere/s Antioxidans/Antioxidantien ein oder mehrere Flavonoide und/oder Coumaranone, wodurch sich deren Schutz gegen gegen UV-Strahlung und/oder oxidativen Stress erheblich verbessert. Gegenstand der Erfindung ist daher auch eine Zubereitung, die **dadurch gekennzeichnet** ist, dass sie eine oder mehrere weitere Verbindung/en, ausgewählt aus der Gruppe der Flavonoide und/oder Coumaranone, enthält.

Als Flavonoide werden die Glykoside von Flavanonen, Flavonen, 3-Hydroxyflavonen (= Flavonolen), Auronen, Isoflavonen und Rotenoiden aufgefaßt [Römpp Chemie Lexikon, Band 9, 1993]. Im Rahmen der vorliegenden Erfindung werden hierunter jedoch auch die Aglykone, d.h. die zuckerfreien Bestandteile, und die Derivate der Flavonoide und der Aglykone verstanden. Weiterhin wird im Rahmen der vorliegenden Erfindung unter dem Begriff Flavonoid auch Anthocyanidin (Cyanidin) verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavonoide sind vorzugsweise ausgewählt aus folgenden Verbindungen: 4,6,3',4'-Tetrahydroxyauron, Quercetin, Rutin, Isoquercetin, Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfaten und Phosphaten. Unter den Flavonoiden sind insbesondere Rutin und Troxerutin bevorzugt. Ganz außerordentlich bevorzugt ist Troxerutin.

Unter den Coumaranonen ist 4,6,3',4'-Tetrahydroxybenzylcoumaranon-3 bevorzugt.

Der Anteil an einem oder der mehreren Antioxidantien in den erfindungsgemässen Zubereitungen, insbesondere in der kosmetischen Formulierung, beträgt vorzugsweise von 0,001 bis 5 Gew.%, besonders bevorzugt von 0,01 bis 2 Gew.% bezogen auf die gesamte Formulierung.

Die erfindungsgemässe Zubereitung kann als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin. P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Formulierungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäße Zubereitung, insbesondere die kosmetische oder pharmazeutische Formulierung, kann weiter als Inhaltsstoff auch Ectoin [(S)-1,4,5,6-Tetrahydro-2-methyl-4-pyrimidincarbonsäure] enthalten und bewirken dann einen Schutz von Zellen der Haut, insbesondere einen Schutz der Langerhanszellen.

Die bereits beschriebene entzündungshemmende Wirkung der erfindungsgemäßen Zubereitung enthaltend [5-Hydroxy-7-methoxy-2-(4'-methoxy-phenyl)-4-oxo-4H-chromen-8yl-]-sulfonsäuremonoester und/oder einen diese Verbindung enthaltenden Extrakt kann weiter verbessert werden, wenn weiterhin ein oder mehrere 1-(2-Hydroxyaryl)-alkan-1-on-oxime (wie z.B. in der EP 0 149 242 beschrieben), vorzugsweise 2-Hydroxy-5-methyl-laurophenonoxim, enthalten ist/sind. Besonders vorteilhaft sind Formulierungen enthaltend [5-Hydroxy-7-methoxy-2-(4'-methoxy-phenyl)-4-oxo-4H-chromen-8yl-]-sulfonsäuremonoester und/oder einen diese Verbindung enthaltenden Extrakt und 2-Hydroxy-5-methyllaurophenonoxim, worin die genannten Substanzen in einem Gewichtsverhältnis von 1:10 bis 10 : 1 enthalten sind. Anwendungsformen derartiger Formulierungen sind z.B. After-Sun-Präparate.

In die erfindungsgemässe Zubereitung, insbesondere in die kosmetische und/oder pharmazeutische Formulierung, können auch weitere Wirkstoffe eingearbeitet werden, z.B.
- Hydroxyectoin [(S,S)-1,4,5,6-Tetrahydro-5-hydroxy-2-methyl-4-pyrimidincarbonsäure]
- Wirkstoffe, die zur Wundbehandlung dienen können, wie z.B. Allantoin
- Insekt-Repellentien wie z.B. 3-[N-n-butyl-N-acetyl]-aminopropionsäureethylester [CAS-Nr. 52304-36-6]
- Sorbit für die Hautpflege [z.B. Karion^{®}F flüssig oder Karion^{®}FP flüssig]
- Biotin
- anti-ageing-Produkte wie z.B. Mischungen enthaltend Hydroxyprolin oder Derivate von Hydroxyprolin, z.B. Mischungen enthaltend Lecithin, Hydroxyprolindipalmitat, Sitosterol, Linolsäure, Tocopherol, Natriumascorbat, Mannit, Phenoxyethanol, Methylparaben, Ethylparaben, Propylparaben, Butylparaben, Wasser [z.B. RonaCare^{™} ASC III^{®}] oder z.B. Mischungen enthaltend Lecithin, hydroxyliertes Lecithin, L-Hydroxyprolin, Dinatrium Rutinyldisulfat, Phenoxyethanol, Mannit, Magnesiumascorbylphosphat, Methylparaben, Ethylparaben, Propylparaben, Butylparaben, Sitosterol, Tocopherol, Natriumascorbat, Wasser [z.B. RonaCare^{™} VTA]
- Bisabolol.

Die [5-Hydroxy-7-methoxy-2-(4'-methoxy-phenyl)-4-oxo-4H-chromen-8yl-]-sulfonsäuremonoester bzw. der diese Verbindung enthaltende Extrakt können in der üblichen Weise in die erfindungsgemässe Zubereitung eingearbeitet werden. Bevorzugt sind Formulierungen für eine äußerliche Anwendung, beispielsweise als Creme, Lotion, Gel, oder als Lösung, die auf die Haut aufgesprüht werden kann. Dabei ist es bevorzugt, wenn die Zubereitung mindestens eine Öl- und mindestens eine Wasser-Ph.ase enthält.

Als Anwendungsformen der erfindungsgemäßen Zubereitungen seien z.B. genannt: Lösungen, Emulsionen, PIT-Emulsionen, Suspensionen, Pasten, Salben, Gele, Cremes, Seifen, tensidhaltige Reinigungspräparate, Lotionen, Öle, Puder, Sprays und Aerosole. Weitere Anwendungsformen sind z.B. Sticks, Shampoos und Duschbäder. Zusätzlich zu [5-Hydroxy-7-methoxy-2-(4'-methoxy-phenyl)-4-oxo-4H-chromen-8yl-]-sulfonsäuremonoester und/oder dem diese Verbindung enthaltende Extrakt können der Formulierung beliebige übliche Trägerstoffe, Hilfsstoffe und gegebenenfalls weitere Wirkstoffe zugesetzt werden.

Vorzuziehende Hilfsstoffe stammen aus der Gruppe der Könservierungsstoffe, Antioxidantien, Stabilisatoren, Lösungsvermittler, Vitamine, Färbemittel, Geruchsverbesserer, Filmbildner, Verdickungsmittel, Feuchthaltemittel.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Die Emulsionen können in verschiedenen Formen vorliegen. So können sie z.B. eine Emulsion oder Mikroemulsion vom Typ Wasser-in-Öl (W/O), oder vom Typ Öl-in-Wasser (O/W), oder eine multiple Emulsion, beispielsweise vom Typ Wasser-in-Öl-in-Wasser (W/O/W), darstellen.

Die Zubereitung ein auch als emulgatorfreie, disperse Formulierung, vorliegen. Sie kann beispielsweise eine Hydrodispersion oder eine Pikkering-Emulsion darstellen.

Die Zubereitung kann auch als PIT-Emulsion oder als Hydrogel vorliegen und auch Liposomen, die beispielsweise Wirkstoffe umschliessen, enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Pasten, Salben, Gele und Cremes können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, lsothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Weitere Anwendungsformen der erfindungsgemäßen Zubereitung sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Alle Verbindungen oder Komponenten, die in der erfindungsgemäßen Zubereitung verwendet werden können, sind entweder bekannt und käuflich erwerbbar oder können nach bekannten Verfahren synthetisiert werden.

Die Formulierung kann Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer [5-Hydroxy-7-methoxy-2-(4'-methoxy-phenyl)-4-oxo-4H-chromen-8yl-]-sulfonsäuremonoester und/oder einen diese Verbindung enthaltenden Extrakt und gegebenenfalls noch weiteren Lichtschutzfiltern Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglycol oder Glycerin, und ein Verdickungsmittel wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die Formulierung kann auch zum Schutz der Haare gegen photochemische Schäden verwendet werden, um Veränderungen von Farbnuancen, ein Entfärben oder Schäden mechanischer Art zu verhindern. Geeignet ist hierbei eine Konfektionierung als Shampoo, Lotion, Gel oder Emulsion zum Ausspülen, wobei die jeweilige Formulierung vor oder nach dem Shamponieren, vor oder nach dem Färben oder Entfärben bzw. vor oder nach der Dauerwelle aufgetragen wird. Es kann auch eine Formulierung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Formulierung kann außer [5-Hydroxy-7-methoxy-2-(4'-methoxy-phenyl)-4-oxo-4H-chromen-8yl-]-sulfonsäuremonoester und/oder einem diese Verbindung enthaltende Extrakt und weiteren UV-Filtern verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäße Zubereitung kann mit Hilfe von Techniken hergestellt werden, die dem Fachmann wohl bekannt sind.

Zum Schutz der Haut und/oder natürlicher oder sensibilisierter Haare vor Sonnenstrahlen wird auf die Haut oder die Haare eine kosmetische Zubereitung, enthaltend [5-Hydroxy-7-methoxy-2-(4'-methoxy-phenyl)-4-oxo-4H-chromen-8yl-]-sulfonsäuremonoester und/oder einen diese Verbindung enthaltender Extrakt aufgetragen. Als sensibilisierte Haare werden dabei Haare verstanden, welche einer chemischen Behandlung, wie einer Dauerwellenbehandlung, einem Färbe- oder Entfärbeprozeß unterzogen worden sind.

Ferner wirken [5-Hydroxy-7-methoxy-2-(4'-methoxy-phenyl)-4-oxo-4H-chromen-8yl-]-sulfonsäuremonoester und/oder der diese Verbindung enthaltende Extrakt auch stabilisierend auf die Formulierung. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist besonders vorteilhaft bei der Zusammensetzungen zum Schutz der Haut gegen die Einwirkung von UV-Strahlen, da diese Kosmetika besonders hohen Belastungen durch die UV-Strahlung ausgesetzt sind.

Die vorteilhaften Eigenschaften von [5-Hydroxy-7-methoxy-2-(4'-methoxyphenyl)-4-oxo-4.H-chromen-8yl-]-sulfonsäuremonoester bzw. des diese Verbindung enthaltenden Extrakts können z.B. auch bei ihrer Verwendung in Nahrungsmitteln oder als Nahrungsergänzungsmittel oder als "functional food" ausgenutzt werden. Beispielsweise können [5-Hydroxy-7-methoxy-2-(4'-methoxy-phenyl)-4-oxo-4H-chromen-8yl-]-sulfonsäuremonoester und/oder der diese Verbindung enthaltende Extrakt die weiterhin in dem Nahrungsmittel, dem Nahrungsergänzungsmittel oder dem "functional food" enthaltenen Verbindungen oder auch den Organismus vor Oxidation bzw. vor der Einwirkung von Radikalen schützen.

Ein Gegenstand der Erfindung ist daher auch ein Nahrungsmittel, das mit [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereichert ist.

Ein weiterer Gegenstand der Erfindung ist ein Nahrungsergänzungsmittel, das [5-Hydroxy-7-methoxy-2-(4'-methoxy-phenyl)4-oxo-4H-chromen-8yl-]-sulfonsäuremonoester und/oder einen diese Verbindung enthaltenden Extrakt enthält. Nahrungsergänzungsmittel sind vorzugsweise Zubereitungen im Sinne der oben stehenden allgemeinen Definition und werden vorzugsweise oral verabreicht.

Die weiteren zu Nahrungsmitteln ausgeführten Erläuterungen gelten sinngemäß auch für Nahrungsergänzungsmittel und für "functional food". Die Nahrungsmittel, die nach der vorliegenden Erfindung mit [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren. Nahrungsmittel, die nach der vorliegenden Erfindung mit [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensauce, Tomatenpüree, usw.. Weitere Beispiele für Nahrungsmittel, die nach der vorliegenden Erfindung [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereichert zu werden, beispielsweise Multivitaminpräparate, Mineralstoffmischungen oder gezuckerter Saft. Als weitere Beispiele für Nahrungsmittel, die nach der vorliegenden Erfindung mit [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereichert werden können, seien Nahrungsmittelzubereitungen, beispielsweise zubereitete Zerealien, Gebäck, Mischgetränke, speziell für Kinder zubereitete Nahrungsmittel wie Joghurt, Diätnahrungsmittel, kalorienarme Nahrungsmittel oder Tierfutter genannt.

Die Nahrungsmittel, die nach der vorliegenden Erfindung mit [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereichert werden können, umfassen somit alle genießbaren Kombinationen von Kohlehydraten, Lipiden, Proteinen, anorganischen Elementen, Spurenelementen, Vitaminen, Wasser und aktiven Metaboliten von Pflanzen und Tieren.

Die Nahrungsmittel, die nach der vorliegenden Erfindung mit [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereichert werden können sowie die Nahrungsergänzungsmittel, die [5-Hydroxy-7-methoxy-2-(4'-methoxy-phenyl)-4-oxo-4H-chromen-8yl-]-sulfonsäuremonoester und/oder einen diese Verbindung enthaltenden Extrakt enthalten, werden vorzugsweise oral angewendet, z.B. in Form von Essen, Pillen, Tabletten, Kapseln, Pulver, Syrups, Lösungen oder Suspensionen.

Wie dargestellt können durch Verwendung/Einarbeitung von [5-Hydroxy-7-methoxy-2-(4'-methoxy-phenyl)-4-oxo-4H-chromen-8yl-]-sulfonsäuremonoester und/oder dem diese Verbindung enthaltenden Extrakt in pharmazeutische und/oder kosmetische Zubereitungen, Nahrungsmittel und/oder Nahrungsergänzungsmittel wertvolle kosmetische Zubereitungen, pharmazeutische Zubereitungen, Nahrungsmittel und/oder Nahrungsergänzungsmittel hergestellt werden können. Gegenstand der Erfindung ist daher auch ausdrücklich die Verwendung von [5-Hydroxy-7-methoxy-2-(4'-methoxy-phenyl)-4-oxo-4H-chromen-8yl-]-sulfonsäuremonoester und/oder einem diese Verbindung enthaltenden Extrakt zur Herstellung einer kosmetischen Zubereitung, einer pharmazeutischen Zubereitung, eines Nahrungsmittels und/oder eines Nahrungsergänzungsmittels.

Soweit die erfindungsgemäße Zubereitung [5-Hydroxy-7-methoxy-2-(4'-methoxy-phenyl)-4-oxo-4H-chromen-8yl-]-sulfonsäuremonoester als Reinsubstanz enthält, ist diese Verbindung, bezogen auf die gesamte Zubereitung, in folgenden Mengen enthalten:
- im Fall, dass die Zubereitung einer kosmetische und/oder pharmazeutische Formulierung ist, in einer Menge von 0.001 bis 100 Gew.-%, vorzugsweise in einer Menge von 0.01 bis 30 Gew.-%, besonders bevorzugt in einer Menge von 0.1 bis 10 Gew.-%
- im Fall, dass die Zubereitung ein Nahrungsmittel ist, in einer Menge von 0.00001 bis 20 Gew.-%, vorzugsweise in einer Menge von 0,001 bis 10 Gew.-%, und
- im Fall, dass die Zubereitung ein Nahrungsergänzungsmittel ist, vorzugsweise von 0.1 bis 80 Gew.-% bezogen auf das gesamte Nahrungsergänzungsmittel.

Soweit die erfindungsgemäße Zubereitung einen [5-Hydroxy-7-methoxy-2-(4'-methoxy-phenyl)-4-oxo-4H-chromen-8yl-]-sulfonsäuremonoester enthaltenden Extrakt enthält, ist dieser, bezogen auf die gesamte Zubereitung, in folgenden Mengen enthalten:
- im Fall, dass die Zubereitung einer kosmetische und/oder pharmazeutische Formulierung ist, in einer Menge von 0.01 bis 100 Gew.-%, vorzugsweise in einer Menge von 0.1 bis 60 Gew.-%, besonders bevorzugt in einer Menge von 1 bis 30 Gew.-%
- im Fall, dass die Zubereitung ein Nahrungsmittel ist, in einer Menge von 0.01 bis 20 Gew.-%,, vorzugsweise in einer Menge von 0.1 bis 10 Gew.-%, und
- im Fall, dass die Zubereitung ein Nahrungsergänzungsmittel ist, vorzugsweise von 0,1 bis 80 Gew.% bezogen auf das gesamte Nahrungsergänzungsmittel.

Die mit [5-Hydroxy-7-methoxy-2-(4'-methoxy-phenyl)-4-oxo-4H-chromen-8yl-]-sulfonsäuremonoester und/oder einem diese Verbindung enthaltenden Extrakt angereicherten Nahrungsmittel können mit Hilfe von Techniken hergestellt werden, die dem Fachmann wohl bekannt sind.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen.

Die vollständige Offenbarung aller vor- und nachstehend aufgeführten Anmeldungen und Veröffentlichungen sind durch Bezugnahme in diese Anmeldung eingeführt.

Alle Verbindungen oder Komponenten, die in den erfindungsgemässen Zubereitungen verwendet werden können, sind entweder bekannt und käuflich erhältlich oder können nach bekannten Methoden synthetisiert werden.

Die INCl-Namen der verwendeten Rohstoffe sind wie folgt (die INCI-Namen werden definitionsgemäß in Englischer Sprache angegeben):

| **Rohstoff** | **INCl-Name** |
|---|---|
| Abil WE 09 | Polyglyceryl-4-Isostearate, Cetyl |
| | Dimethicone Copolyol, Hexyl |
| | Laurate |
| Antaron V-220 | PVP/Eicosene Copolymer |
| Arlacel 80 | Sorbitan Oleate |
| Arlacel 165 V | Glyceryl Stearate, PEG-100 |
| | Stearate |
| Avocadoöl | Persea Gratissima |
| Bienenwachs | Beeswax |
| Biobase^{™} EP | Glyceryl Stearate, Cetearyl Alcohol, |
| | Sodium Stearoyl Lactylate, Lecithin |
| Carbopol ETD 2050 | Carbomer |
| Cetiol V | Decyl Oleate |
| Cetylalkohol | Cetyl Alcohol |
| Cetylisononanoat | Cetyl Isononanoate |
| Cutina HR | Hydrogenated Castor Oil |
| Dimeticon | Dimethicone |
| Eusolex^{®}232 | Phenylbenzimidazole Sulfonic Acid |
| Eusolex^{®} 2292 | Octyl Methoxycinnamate, BHT |
| Eusolex^{®} 6300 | 4-Methylbenzylidene Camphor |
| Eusolex 8300 | 4-Methylbenzylidene |
| Eusolex^{®} 9020 | Butyl Methoxydibenzoylmethane |
| Eusolex^{®}HMS | Homosalate |
| Eusolex T-Aqua | Aqua (Water), Titanium Dioxide, |
| | Alumina, Sodium Metaphosphate, |
| | Phenoxyethanol, Sodium Methyl- |
| | paraben |
| Eutanol G | Octyldodecanol |
| Germaben II | Propylene Glycol, Diazolidinyl |
| | Urea, Methylparaben, |
| | Propylparaben |
| Germaben II-E | Propylene Glycol, Diazolidinyl |
| | Urea, Methylparaben, |
| | Propylparaben |
| Glycerin | Glycerin |
| Glycerin (87%) | Glycerin |
| Glycerin (87% reinst) | Glycerin |
| Glycerin, wasserfrei | Glycerin |
| Hetester PHA | Propylene Glycol Isoceteth-3 |
| | Acetate |
| Hexyllaurat | Hexyl Laurate |
| Imwitor 960 K Schuppen | Glyceryl Stearate SE |
| Isolan PDI | Diisostearoyl Polyglyceryl-3- |
| | Diisostearat |
| Isopropylmyristat | Isopropyl Myristate |
| Isopropylpalmitat | Isopropyl Palmitate |
| Jojobaöl | Buxus Chinensis (Jojoba Oil) |
| Karion F flüssig | Sorbitol |
| Keltrol RD | Xanthan Gum |
| Magnesiumsulfat | Magnesium Sulfate |
| Magnesiumsulfat-Heptahydrat | Magnesium Sulfate |
| Methyl-4-hydroxybenzoat | Methylparaben |
| Miglyol 812 | Caprylic/Capric Triglyceride |
| Miglyol 812 N | Caprytic/Capric Triglyceride |
| Miglyol 812, Neutralöl | Caprylic/Capric Triglyceride |
| Mirasil CM5 | Cyclomethicone |
| Mirasil DM 350 | Dimethicone |
| Montanov 68 | Cetearyl Alcohol, Cetearyl |
| | Glucoside |
| Natriumchlorid | Sodium Chloride |
| Natronlauge, 10%ig | Sodium Hydroxide |
| Oxynex^{®}K | PEG-8, Tocopherol, Ascorbyl |
| | Palmitate, Ascorbic Acid, Citric Acid |
| Panthenol-D | Panthenol |
| Paracera M | Microwax |
| Paraffinöl, fl. | Mineral Oil |
| Parfümöl TND-2417 | Parfum |
| Pemulen TR-1 | Acrylates/C₁₀₋₃₀ Alkyl Acrylate |
| | Crosspolymer |
| Pemulen^{®} TR-2 | Acrylates/C₁₀₋₃₀ Alkyl Acrylate |
| | Crosspolymer |
| Performa^{®} V 825 | Synthetic Wax |
| Polyglyceryl-2-Dipolyhydroxy- | Polyglyceryl-2 Dipolyhydroxystearate |
| stearat | |
| Prisorine 2021 | Isopropyl Isostearate |
| Propandiol-1,2 | Propylene Glycol |
| Propyl-4-hydroxybenzoat | Propylparaben |
| Rhodicare S | Xanthan Gum |
| RonaCare^{™} ASC III | Aqua, Lecithin, Dipalmitoyl |
| | Hydroxyproline, Phenoxyethanol, |
| | Tall Oil Sterol, Linoleic Acid, |
| | Tocopherol, Sodium Ascorbate, |
| | Mannitol, Methylparaben, |
| | Ethylparaben, Propylparaben, |
| | Butylparaben |
| RonaCare^{™} Bisabolol | Bisabolol |
| RonaCare^{™} Ectoin | Ectoin |
| RonaCare^{™} LPO | Lauryl p-Cresol Ketoxime |
| RonaCare^{™} Tocopherolacetat | Tocopheryl Acetate |
| Sepigel 305 | Polyacrylamide, C₁₃₋₁₄ Isoparaffin, |
| | Laureth-7 |
| SFE 839 | Cyclopentasiloxane, Dimethicone/ |
| | Vinyldimethicone Crosspolymer |
| Shea Butter | Shea Butter |
| Steareth-2 | Steareth-2 |
| Steareth-10 | Steareth-10 |
| Stearinsäure | Stearic Acid |
| DL-α-Tocopherolacetat | Tocopherol Acetate |
| Triethanolamin | Triethanolamine |
| Triethanolamin reinst | Triethanolamine |
| Wasser, demineralisiert | Aqua (Water) |
| Zinkstearat | Zinc Stearate |

Die Beispiele, ohne hierauf beschränkt zu sein, erläutern die Erfindung.

### Beispiel 1

### Nachweis der antientzündlichen Aktivität

Die antientzündlichen Eigenschaften von [5-Hydroxy-7-methoxy-2-(4'-methoxy-phenyl)-4-oxo-4H-chromen-8yl-]-sulfonsäuremonoester sind nachweisbar im Keratinozyten-Monoschicht-PGE₂-Modell. Zur Induktion der Entzündung werden Keratinozyten 24 Stunden in 96-Well-Platten (15.000 Zellen/Well) mit der pro-inflammatorisch wirkenden Substanz Phorbolmyristatacetat (PMA) inkubiert. Die vorinkubierten Zellen (0,1 µg/ml Endkonzentration PMA) werden 24 Stunden mit 10⁻⁶ M Indometacin (Positivkontrolle) bzw. 0,2 mM [5-Hydroxy-7-methoxy-2-(4'-methoxyphenyl)-4-oxo-4H-chromen-8yl-]-sulfonsäuremonoester inkubiert. Als Negativkontrolle dient eine Kontrollkultur, die kein PMA enthält. Nach Inkubation wird in allen drei Proben der Gehalt an Prostaglandin E₂ (PGE₂) durch ELISA Kits bestimmt.

### Beispiel 2

### Extraktion

10 kg Blattmaterial von Sidastrum micranthum werden mit 120 Liter Ethanol 2x in der Hitze extrahiert. Die vereinigten Auszüge werden auf 10 Liter eingeengt, mit 5 kg Eis und 5 Liter kaltem Wasser bei unter 10°C für 2 Stunden gerührt, und filtriert. Das Filtrat (20 Liter) wird auf 8 Liter unter Hitze unter vermindertem Druck eingeengt.
Nach dem Abkühlen unter 25°C wird abfiltriert. Das erhaltene Filtrat wird bis zu einem Gesamtfeststoffgehalt von 10% weiter eingeengt und bei 35°C abgefiltert um Begleitflavonoid zu entfernen. Das erhaltene Filtrat wird weiter eingeengt bis zu einem Gesamtfeststoffgehalt von 20% und bei 35°C abgefiltert. Der erhaltene Kuchen mit [5-Hydroxy-7-methoxy-2-(4'-methoxy-phenyl)-4-oxo-4H-chromen-8yl-]-sulfonsäuremonoester wird mit 70% Ethanol gewaschen. Das Filtrat wird zur Ausbeuteerhöhung bis 45% Feststoffgehalt konzentriert. Nach Erreichen der Raumtemperatur wird abgefiltert, und der Kuchen mit [5-Hydroxy-7-methoxy-2-(4'-methoxyphenyl)-4-oxo-4H-chromen-8yl-]-sulfonsäuremonoester ebenfalls mit 70% Ethanol gewaschen.
[5-Hydroxy-7-methoxy-2-(4'-methoxy-phenyl)-4-oxo-4H-chromen-8yl-]-sulfonsäuremonoester wird in einer Ausbeute von 1,2 Gew.-% bezogen auf die getrockneten Blätter erhalten.
¹³C NMR Daten:
δ [ppm] (in DMSO): 55,43 (OCH₃), 56,38 (OCH₃), 95,90 (C-6), 102,70 (C-3); 103,69 (C-9), 114,27 (C-5',C-3'),122,58 (C-8), 123,00 (C-1'), 129.06 (C-2-, C-6'), 149,33 (C-10), 156,93 (C-5), 159,02 (C-7), 162,29 (C-4'), 163,92 (C-2), 182,13 (C=O)
^{1H} NMR Daten:
δ [ppm] , 3,90 (s, 3H, CH₃) 3,95 (s, 3H, CH₃) 6,55 (s,1 H, H-6), 6,85 (s, 1H, H-3), 7,1 (d , 2H, H-5', H-3'), 8,1 (d, 2H, H-2',H6'), 12,9 (s, 1 H, OH).

### Massenspektrum

Maldi/MS: 824,9 (2M+K), 392,9 (M-H)⁻, 152,9

### UV-Absorbtionsspektum

Konzentration: 1mg/100ml

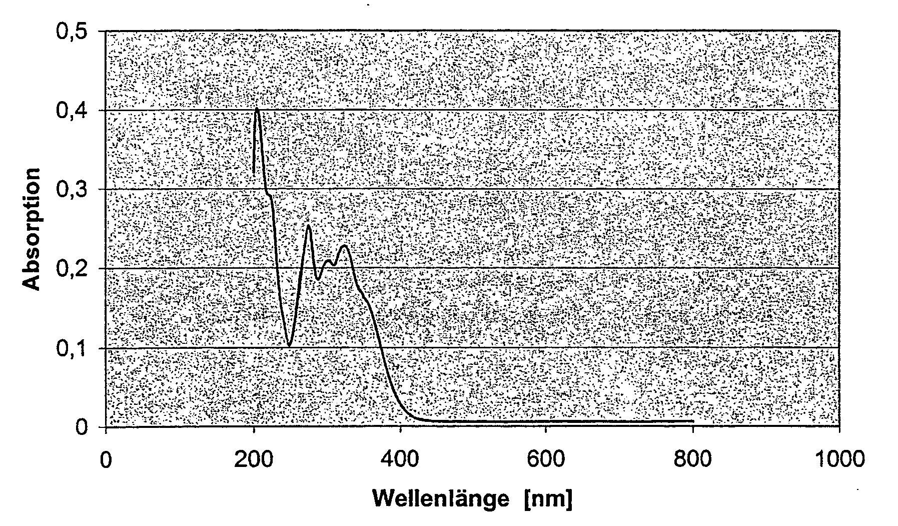

### Assays

### Expression des Tansglutaminase-Gens

In Zellkulturen (menschliche Keratinozyten) wird das Kulturmedium durch Kulturmedium mit (Test) bzw. ohne Testsubstanz (Kontrolle) ersetzt. Die Zellen werden eine definierte Zeit weiterkultiviert, geerntet und bei -80° eingefroren.
Die RNA wird extrahiert, wobei jede Kultur ca. 200 µg RNA enthält. Die Lösung wird auf 1µg/µl RNA eingestellt und mit DNAse I behandelt, um alle DNA-Reste zu entfernen. Die RNA Menge wird auf 2 µg/ml eingestellt. Die mRNA wird auf cDNA ³²P-markierte Proben umgeschrieben und mittels Chromatographie aufgereinigt.
Die DNA Sequenzen werden auf cDNA Chip-Membranen immobilisiert und hybridisiert (über Nacht bei 68°C). Die Membranen werden intensiv gewaschen und die Radioaktivität jedes Messpunkts gemessen. Zunahme der Radioaktivität bedeutet eine Aufregulierung der entsprechenden RNA verglichen mit der Kontrolle.

### Bestimmung der transglutaminase mittels PCR (Polymerase Chain reaction

Menschliche Keratinozyten werden mit der Testsubstanz inkubiert und die RNA mit Tri-Reagent extrahiert. Die RNA der Transglutaminase wird mit biotinylated oligo(dT) und Superscript II reverse transcriptase transkribiert. Die Messung erfolgt mit einem LightCycler (Roche), wobei die Fluoreszenz während den PCR Zyclen andauernd gemessen wird. Aus der Relation von "Fluoreszenz" und der Nummer der PCR Zylusses ergibt sich die relative Expression der Transglutaminase RNA.

### Transglutaminase Enzymaktivität in Zellkulturen

Menschliche Keratinozyten werden kultiviert. Eine Probe wird mit 50 µM Testsubstanz inkubiert und 96 Stunden kultiviert. Als Positivkontrolle wird 1.5mM CaCl₂ verwendet, als Negativkontrolle 1µM Retinol. Eine unbehandelte Probe dient als Kontrolle. TGk wird aus den Zellen extrahiert. Die Enzymaktivität wird durch kovalente Bindung von ³H-Putrescin an Kasein gemessen. Kasein wird mit Trichloressisäure gefällt und nach Aufreinigung und Trocknung mittels Flüssigszintillation vermessen.

### Beispiel 3

### Lotion (W/O) zum Auftragen auf die Haut

| | | **Gew.-%** |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | [5-Hydroxy-7-methoxy-2-(4'-methoxy-phenyl)-4-oxo- | |
| | 4H-chromen-6yl-]-sulfonsäuremonoester | 0,5 |
| | | |
| **B** | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

### Beispiel 4

### Lotion (W/O) zum Auftragen auf die Haut

| | | **Gew.%** |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | | |
| **B** | [5-Hydroxy-7-methoxy-2-(4'-methoxy-phenyl)-4-oxo- | |
| | 4H-chromen-6yl-]-sulfonsäuremonoester | 1,0 |
| | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

### Beispiel 5

### Lotion (W/O) zum Auftragen auf die Haut

| | | **Gew.%** |
|---|---|---|
| **A** | 4,6,3`,4`-Tetrahydroxybenzyicoumaranon-3 | 1,0- |
| | Polyglyceryl-2-Dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | [5-Hydroxy-7-methoxy-2-(4'-methoxy-phenyl)-4-oxo- | |
| | 4H-chromen-6yl-]-sulfonsäuremonoester | 1,0 |
| | | |
| **B** | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

### Beispiel 6

Aus folgenden Komponenten wird eine Creme (O/W), enthaltend Ectoin, hergestellt:

| | | | **Gew.%** |
|---|---|---|---|
| **A** | Paraffin, dünnflüssig | (1) | 8,0 |
| | Isopropylmyristat | (1) | 4,0 |
| | Mirasil CM5 | (2) | 3,0 |
| | Stearinsäure | (1) | 3,0 |
| | Arlacel 165 V | (3) | 5,0 |
| [5-Hydroxy-7-methoxy-2-(4'-methoxy-phenyl)-4-oxo-H-chromen-6yl-]-sulfonsäuremonoester | | | 1,0 |
| | | | |
| **B** | Glycerin (87%) | (1) | 3,0 |
| | Germaben II | (4) | 0,5 |
| | Wasser, demineralisiert | | ad 100 |
| | | | |
| **C** | RonaCare^{™} Ectoin | (1) | 1,0 |

### Herstellung

Zunächst werden die Phasen A und B getrennt auf 75°C erwärmt. Danach wird Phase A unter Rühren langsam zu Phase B gegeben und solange gerührt, bis eine homogene Mischung entsteht. Nach Homogenisierung der Emulsion wird unter Rühren auf 30°C abgekühlt. Anschließend wird auf 35°C erwärmt, die Phase C zugegeben und bis zur Homogenität gerührt.

### Bezugsquellen

(1) Merck KGaA
(2) Rhodia
(3) Uniqema
(4) ISP

### Beispiel 7

### Topische Zusammensetzung als W/O-Emulsion

| | | | **Gew.%** |
|---|---|---|---|
| **A** | Isolan PDI | (2) | 3,0 |
| | Paraffinöl, fl. | (1) | 17,0 |
| | Isopropylmyristat | | 5,0 |
| | Bienenwachs | | 0,2 |
| | Cutina HR | (2) | 0,3 |
| | [5-Hydroxy-7-methoxy-2-(4'-methoxy-phenyl)-4-oxo-H-chromen-6yl-]-sulfonsäuremonoester | | 1,0 |
| | | | |
| **B** | Wasser, demineralisiert | | ad 100 |
| | Glycerin (87%) | | 4,0 |
| | Magnesiumsulfat | | 1,0 |
| | Germaben II-E | (3) | 1,0 |
| | | | |
| **C** | RonaCare^{™} LPO | (1) | 2,0 |

### Herstellung

Die Phasen A und B werden auf 75°C erwärmt. Phase B wird unter Rühren zu Phase A gegeben. Anschließend wird das Gemisch bei 9000upm 2 Min. mit dem Turrax homogenisiert. Das erhaltene Gemisch wird auf 30 bis 35°C abgekühlt, und C wird eingerührt.

### Bezugsquellen

(1) Merck KGaA
(2) Goldschmidt AG
(3) ISP

## Patentansprüche

1. [5-Hydroxy-7-methoxy-2-(4'-methoxy-phenyl)-4-oxo-4H-chromen-8yl-]-sulfonsäuremonoester, dessen physiologisch unbedenkliche Salze und Solvate.

2. Extrakt enthaltend [5-Hydroxy-7-methoxy-2-(4'-methoxy-phenyl)-4-oxo-4H-chromen-8yl-]-sulfonsäuremonoester, dessen physiologisch unbedenkliche Salze und Solvate, erhältlich durch Extraktion von Pflanzenmaterial, ausgewählt aus Sidastrum acuminatum, Sidastrum burrerense, Sidastrum E.G. Baker, Sidastrum kicranthum, Sidastrum lodiegense, Sidastrum multiflorum, Sidastrum micranthum, Sidastrum paniculatum, Sidastrum strictum, Sidastrum tehuacanum oder Sidastrum quinquenervium.

3. Extrakt nach Anspruch 2, erhältlich durch Extraktion von Pflanzenmaterial der Art Sidastrum micranthum.

4. Zubereitung mindestens enthaltend die Verbindung nach Anspruch 1 und/oder einen Extrakt nach Anspruch 2 oder 3 sowie gegebenenfalls Träger- und/oder Hilfsstoffe.

5. Zubereitung nach Anspruch 4, **dadurch gekennzeichnet, dass** diese ein Arzneimittel ist.

6. Zubereitung nach Anspruch 4, **dadurch gekennzeichnet, dass** diese ein kosmetisches Mittel ist.

7. Zubereitung nach Anspruch 5 oder 6, **dadurch gekennzeichnet, dass** diese ein Hautbehandlungsmittel ist.

8. Zubereitung zur topischen Anwendung umfassend
a) die Verbindung nach Anspruch 1 und/oder einen Extrakt nach Anspruch 2 oder 3,
b) einen hautverträglichen Träger, und
c) optional einen oder mehrere weitere Wirkstoffe mit hautpflegender und/oder entzündungshemmender Wirkung.

9. Zubereitung nach Anspruch 7 und 8, **dadurch gekennzeichnet, dass** sie weiterhin einen oder mehrere UV-Filter enthält.

10. Zubereitung nach einem oder mehreren der Ansprüche 4 bis 9, **dadurch gekennzeichnet, dass** sie ein oder mehrere weitere/s Antioxidans/Antioxidantien enthält.

11. Zubereitung nach einem oder mehreren der Ansprüche 4 bis 10, **dadurch gekennzeichnet, dass** eine oder mehrere weitere Verbindung/en, ausgewählt aus der Gruppe der Flavonoide und/oder Coumaranone, enthält.

12. Nahrungsmittel, **dadurch gekennzeichnet, dass** dieses mit der Verbindung nach Anspruch 1 und/oder einem Extrakt nach Anspruch 2 oder 3 angereichert ist.

13. Nahrungsergänzungsmittel, **dadurch gekennzeichnet, dass** dieses die Verbindung nach Anspruch 1 und/oder einem Extrakt nach Anspruch 2 oder 3 enthält.

14. Verwendung der Verbindung nach Anspruch 1 oder eines Extrakts nach Anspruch 2 oder 3 zur Herstellung einer kosmetischen Zubereitung, einer pharmazeutischen Zubereitung, eines Nahrungsmittels nach Anspruch 11 und/oder eines Nahrungsergänzungsmittels.

15. Verwendung der Verbindung nach Anspruch 1 und/oder des Extrakts nach Anspruch 2 oder 3 zur Herstellung einer pharmazeutischen und/oder kosmetischen Zubereitung zur Erhöhung der Widerstandskraft der Haut gegenüber Umwelteinflüssen, insbesondere Austrocknung, zur Vermeidung der Hautalterung, zur Verbesserung der Hautstruktur, insbesondere zur Bildung glatter Haut.

16. Verwendung der Verbindung nach Anspruch 1 und/oder des Extrakts nach Anspruch 2 oder 3 zur Herstellung einer pharmazeutischen und/oder kosmetischen Zubereitung zum Schutz gegen oxidativen Stress sowie zur Bekämpfung von Allergien, Entzündungen und/oder Irritationen.

17. Verfahren zur Herstellung der Verbindung nach Anspruch 1 durch Extraktion von Pflanzenmaterial der Gattungen Sidastrum acuminatum, Sidastrum-burrerense, Sidastrum E.G. Baker, Sidastrum-kicranthum, Sidastrum lodiegense, Sidastrum multiflorum, Sidastrum micranthum, Sidastrum paniculatum, Sidastrum strictum, Sidastrum tehuacanum oder Sidastrum quinquenervium.

18. Verfahren nach Anspruch 17, **dadurch gekennzeichnet, dass** als Pflanzenmaterial Sidastrum micranthum Verwendung findet.

## Claims

1. [5-Hydroxy-7-methoxy-2-(4'-methoxyphenyl)-4-oxo-4H-chromen-8-yl]-sulfonic acid monoester, physiologically acceptable salts and solvates thereof.

2. Extract comprising [5-hydroxy-7-methoxy-2-(4'-methoxyphenyl)-4-oxo-4H-chromen-8-yl]sulfonic acid monoester, physiologically acceptable salts and solvates thereof, obtainable by extraction of plant material selected from Sidastrum acuminatum, Sidastrum burrerense, Sidastrum E.G. Baker, Sidastrum kicranthum, Sidastrum lodiegense, Sidastrum multiflorum, Sidastrum micranthum, Sidastrum paniculatum, Sidastrum strictum, Sidastrum tehuacanum or Sidastrum quinquenervium.

3. Extract according to Claim 2, obtainable by extraction of plant material of the species Sidastrum micranthum.

4. Preparation at least comprising the compound according to Claim 1 and/or an extract according to Claim 2 or 3 and optionally excipients and/or adjuvants.

5. Preparation according to Claim 4, **characterised in that** it is a medicament.

6. Preparation according to Claim 4, **characterised in that** it is a cosmetic composition.

7. Preparation according to Claim 5 or 6, **characterised in that** it is a skin-treatment composition.

8. Preparation for topical use comprising
a) the compound according to Claim 1 and/or an extract according to Claim 2 or 3,
b) a skin-tolerated vehicle, and
c) optionally one or more further active ingredients having a skin-care and/or inflammation-inhibiting action.

9. Preparation according to Claim 7 or 8, **characterised in that** it furthermore comprises one or more UV filters.

10. Preparation according to one or more of Claims 4 to 9, **characterised in that** it comprises one or more further antioxidant(s).

11. Preparation according to one or more of Claims 4 to 10, **characterised in that** it comprises one or more further compound(s) selected from the group of the flavonoids and/or coumaranones.

12. Food, **characterised in that** it is enriched with the compound according to Claim 1 and/or an extract according to Claim 2 or 3.

13. Food supplement, **characterised in that** it comprises the compound according to Claim 1 and/or an extract according to Claim 2 or 3.

14. Use of the compound according to Claim 1 or an extract according to Claim 2 or 3 for the preparation of a cosmetic preparation, a pharmaceutical preparation, a food according to Claim 12 and/or a food supplement.

15. Use of the compound according to Claim 1 and/or the extract according to Claim 2 or 3 for the preparation of a pharmaceutical and/or cosmetic preparation for increasing the resistance of the skin to environmental influences, in particular drying out, for preventing skin ageing, for improving the skin structure, in particular for the formation of smooth skin.

16. Use of the compound according to Claim 1 and/or the extract according to Claim 2 or 3 for the preparation of a pharmaceutical and/or cosmetic preparation for protection against oxidative stress and for combating allergies, inflammation and/or irritation.

17. Process for the preparation of the compound according to Claim 1 by extraction of plant material of the species Sidastrum acuminatum, Sidastrum-burrerense, Sidastrum E.G. Baker, Sidastrum-kicranthum, Sidastrum lodiegense, Sidastrum multiflorum, Sidastrum micranthum, Sidastrum paniculatum, Sidastrum strictum, Sidastrum tehuacanum or Sidastrum quinquenervium.

18. Process according to Claim 17, **characterised in that** the plant material used is Sidastrum micranthum.

## Revendications

1. Monoester de l'acide [5-hydroxy-7-méthoxy-2-(4'-méthoxyphényl)-4-oxo-4H-chromén-8-yl]sulfonique, et ses sels et solvats physiologiquement acceptables.

2. Extrait comprenant le monoester de l'acide [5-hydroxy-7-méthoxy-2-(4'-méthoxyphényl)-4-oxo-4H-chromén-8-yl]sulfonique, et ses sels et solvats physiologiquement acceptables, pouvant être obtenu par extraction d'une matière végétale sélectionnée parmi Sidastrum acuminatum, Sidastrum burrerense, Sidastrum E.G. Baker, Sidastrum kicranthum, Sidastrum lodiegense, Sidastrum multiflorum, Sidastrum micranthum, Sidastrum paniculatum, Sidastrum strictum, Sidastrum tehuacanum ou Sidastrum quinquenervium.

3. Extrait selon la revendication 2, pouvant être obtenu par extraction d'une matière végétale issue de l'espèce Sidastrum micranthum.

4. Préparation au moins comprenant le composé selon la revendication 1 et/ou un extrait selon la revendication 2 ou 3 et éventuellement des excipients et/ou adjuvants.

5. Préparation selon la revendication 4, **caractérisée en ce qu'**il s'agit d'un médicament.

6. Préparation selon la revendication 4, **caractérisée en ce qu'**il s'agit d'une composition cosmétique.

7. Préparation selon la revendication 5 ou 6, **caractérisée en ce qu'**il s'agit d'une composition de traitement cutané.

8. Préparation pour une utilisation topique, comprenant
a) le composé selon la revendication 1 et/ou un extrait selon la revendication 2 ou 3,
b) un véhicule toléré par la peau, et
c) éventuellement un ou plusieurs ingrédients actifs supplémentaires ayant une action de soin de la peau et/ou anti-inflammatoire.

9. Préparation selon la revendication 7 ou 8, **caractérisée en ce qu'**elle comprend en outre un ou plusieurs filtres UV.

10. Préparation selon l'une ou plusieurs parmi les revendications 4 à 9, **caractérisée en ce qu'**elle comprend un ou plusieurs antioxydants supplémentaires.

11. Préparation selon l'une ou plusieurs parmi les revendications 4 à 10, **caractérisée en ce qu'**elle comprend un ou plusieurs composés supplémentaires sélectionnés parmi le groupe constitué par les flavonoïdes et/ou les coumaranones.

12. Aliment, **caractérisé en ce qu'**il est enrichi par le composé selon la revendication 1 et/ou par un extrait selon la revendication 2 ou 3.

13. Complément alimentaire, **caractérisé en ce qu'**il comprend le composé selon la revendication 1 et/ou un extrait selon la revendication 2 ou 3.

14. Utilisation du composé selon la revendication 1 ou d'un extrait selon la revendication 2 ou 3, pour la préparation d'une préparation cosmétique, d'une préparation pharmaceutique, d'un aliment selon la revendication 12 et/ou d'un complément alimentaire.

15. Utilisation du composé selon la revendication 1 et/ou de l'extrait selon la revendication 2 ou 3, pour la préparation d'une préparation pharmaceutique et/ou cosmétique destinée à augmenter la résistance de la peau aux influences environnementales, en particulier le dessèchement cutané, à empêcher le vieillissement cutané, à améliorer la structure cutanée, en particulier pour la formation d'une peau lisse.

16. Utilisation du composé selon la revendication 1 et/ou de l'extrait selon la revendication 2 ou 3, pour la préparation d'une préparation pharmaceutique et/ou cosmétique destinée à protéger contre le stress oxydatif et à lutter contre les allergies, les inflammations et/ou les irritations.

17. Procédé de préparation du composé selon la revendication 1, par extraction d'une matière végétale issue des espèces Sidastrum acuminatum, Sidastrum-burrerense, Sidastrum E.G. Baker, Sidastrum-kicranthum, Sidastrum lodiegense, Sidastrum multiflorum, Sidastrum micranthum, Sidastrum paniculatum, Sidastrum strictum, Sidastrum tehuacanum ou Sidastrum quinquenervium.

18. Procédé selon la revendication 17, **caractérisé en ce que** la matière végétale utilisée est Sidastrum micranthum.
